# EUROPEAN PATENT APPLICATION

(11) **EP 4 564 372 A1**
(43) Date of publication of application: **04.06.2025**
(21) Application number: 23847007.4
(22) Date of filing: 27.07.2023
(51) Int. Cl.: G16H 50/70, G16H 50/50, G16H 50/20, G16H 50/30, G16H 30/20, G16H 10/60, G06V 10/764, G06V 10/98, A61B 5/346, A61B 5/00

(54) **METHOD, PROGRAM, AND DEVICE FOR UPDATING ARTIFICIAL INTELLIGENCE MODEL FOR ELECTROCARDIOGRAM READING**

(30) Priority: 29.07.2022 KR 20220094606; 26.07.2023 KR 20230097255
(71) Applicant: Medical AI Co., Ltd., Seoul 06180 (KR)
(72) Inventor: KWON, Joonmyoung, Seoul 06180 (KR); JO, Yongyeon, Seoul 06180 (KR); LIM, Seonyu, Seoul 06180 (KR)
(74) Representative: Patentanwaltskanzlei Matschnig & Forsthuber OG
(86) International application number: PCT/KR2023/010883
(87) International publication number: WO 2024/025350

(57) **Abstract**

The present disclosure is directed to a method, program, and device of updating an artificial intelligence model for electrocardiogram reading. The method is performed by a computing device including at least one processor. The method includes: obtaining electrocardiogram data; classifying the relative value of the electrocardiogram data using at least one of a first value determination, a second value determination, and a third value determination based on preset reference value ranges by analyzing the electrocardiogram data; and combining pieces of electrocardiogram data whose relative values have been classified to re-train a previously constructed first machine learning model and to train one or more new second machine learning models.

## Description

### Technical Field

The present disclosure relates to a method of updating an artificial intelligence model for electrocardiogram reading, and more particularly, to a method capable of advancing an artificial intelligence model for electrocardiogram reading by using electrocardiogram data classified by relative value.

### Background Art

Electrocardiograms (ECGs) are signals that enable the presence or absence of diseases to be determined by measuring electrical signals generated from the hearts and checking for abnormalities in conduction systems each extending from the heart to electrodes.

Such electrocardiograms are measured by electrocardiogram measurement devices and are used as auxiliary tools to measure patients' health states. In general, electrocardiogram measurement devices only show measurement results, and diagnosis is entirely the responsibility of doctors.

Currently, research is being conducted to diagnose diseases rapidly and accurately using artificial intelligence based on electrocardiograms in order to reduce the dependence on doctors. In addition, with the development of wearable and lifestyle electrocardiogram measurement devices, the possibility of diagnosing and monitoring not only heart disease but also various other diseases based on electrocardiograms is emerging.

General clinics that do not have specialized knowledge of electrocardiograms or medical institutions that have difficulty reading electrocardiograms request the electrocardiogram reading of the electrocardiogram data of subjects of measurement from electrocardiogram reading centers. Such an electrocardiogram reading center provides a service that obtains primary reading results using an artificial intelligence model and secondary reading results from specialized medical staff and then transmits the reading result values of a subject of measurement using the primary and secondary reading results back to the medical institution that requested the reading. In this case, the electrocardiogram reading center stores the electrocardiogram data and reading result values in a cumulative manner.

The reading results for the electrocardiogram include the results of the secondary reading from an expert having a high level of medical knowledge, and thus, the reading results for the electrocardiogram have the value of improving the performance of an artificial intelligence model.

Meanwhile, when an artificial intelligence model that has been developed by adding new training data is updated using training data having a high value, it may have a significant impact on the system stability of the operation center that provides the electrocardiogram reading service. However, in the case where an artificial intelligence model is re-trained using newly obtained electrocardiogram data as training data when the reliability of the newly obtained electrocardiogram data is not guaranteed, there is a problem in that the performance of the artificial intelligence model cannot be guaranteed to be superior to that of the artificial intelligence model before the re-training.

Therefore, when the operation center that provides the electrocardiogram reading service re-trains an artificial intelligence model or trains a new artificial intelligence model by randomly using obtained electrocardiogram data as training data, the performance of an artificial intelligence model cannot be guaranteed, and rather, an artificial intelligence model having reduced performance may be installed in the system of the electrocardiogram reading center. Furthermore, a problem arise in that an artificial intelligence model may be overloaded when cumulative electrocardiogram data is used as training data to re-train the artificial intelligence model every time.

### Disclosure

### Technical Problem

The present disclosure has been conceived in response to the above-described background art, and an object of one embodiment of the present disclosure is to provide a method of updating an artificial intelligence model for electrocardiogram reading that enables artificial intelligence models to be re-trained or trained using highly reliable electrocardiogram data, classified by relative value, out of a large amount of cumulative electrocardiogram data and also enables a model having the highest output accuracy to be selected from among the re-trained or trained artificial intelligence models.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method of updating an artificial intelligence model for electrocardiogram reading, the method being performed by a computing device including at least one processor, the method including: obtaining electrocardiogram data; classifying the relative value of the electrocardiogram data using at least one of a first value determination, a second value determination, and a third value determination based on preset reference value ranges by analyzing the electrocardiogram data; and combining pieces of electrocardiogram data whose relative values have been classified to re-train a previously constructed first machine learning model and to train one or more new second machine learning models.

Alternatively, the method may further include selecting a machine learning model having the highest output accuracy as a model for an electrocardiogram reading service by comparing the output accuracy of the re-trained first machine learning model and the output accuracies of the trained second machine learning models.

Alternatively, the method may further include providing an alarm function including information about the machine learning model having the highest output accuracy so that an operator of the electrocardiogram reading service can update the model for the electrocardiogram reading service.

Alternatively, the selection of the model for the electrocardiogram reading service may be performed when a preset selection trigger condition is satisfied.

Alternatively, the selection triggering condition may be any one of a case where the number of machine learning models whose output accuracy exceeds a first criterion exceeds a second criterion and a case where the number of pieces of electrocardiogram data determined to have a disease exceeds a third criterion.

Alternatively, the first value determination may include a first value analysis process of determining the value of the electrocardiogram data by comparing expert reading information read by an expert terminal with electrocardiogram reading information read via the first machine learning model.

Alternatively, the first value analysis process may include: a process of determining whether the electrocardiogram reading information read via the first machine learning model is incorrect; and a process of classifying the relative value of the electrocardiogram data depending on whether the electrocardiogram reading information is incorrect.

Alternatively, the process of classifying the relative value of the electrocardiogram data depending on whether the electrocardiogram reading information is incorrect may include: when the electrocardiogram reading information is incorrect, classifying the electrocardiogram data as a first group having a high relative value; and, when the electrocardiogram reading information is not incorrect, classifying the electrocardiogram data as a second group having a low relative value.

Alternatively, the process of classifying the relative value of the electrocardiogram data depending on whether the electrocardiogram reading information is incorrect may include, when the electrocardiogram reading information is not incorrect and electrocardiogram reading is impossible due to an error signal caused by a noise signal or missing value included in the electrocardiogram data, discarding the electrocardiogram data not to be used as data for re-training or training.

Alternatively, the second value determination may include a second value analysis process that determines the value of the electrocardiogram data by analyzing whether a subject from whom the electrocardiogram data was measured has a disease.

Alternatively, the second value analysis process may include, based on expert reading information read by an expert terminal: when it is determined that the subject from whom the electrocardiogram data was measured has a disease, classifying the electrocardiogram data as a first group having a high relative value; and, when it is determined that the subject from whom the electrocardiogram data was measured does not have a disease, classifying the electrocardiogram data as a second group having a low relative value.

Alternatively, the third value determination may include a third value analysis process that determines the value of the electrocardiogram data based on a tag included in the electrocardiogram data.

Alternatively, the third value analysis process may include: extracting one or more tags from the electrocardiogram data through a tag interface for analyzing tags included in the electrocardiogram data; recognizing at least one tagged information out of the hospital size, the hospital type, the patient type, and the electrocardiogram measurement device type based on the extracted tags; and classifying the relative value of the electrocardiogram data based on the recognized information.

Alternatively, classifying the relative value of the electrocardiogram data based on the recognized information may include: when the hospital size meets a preset reference size or the patient's property matches a preset reference property based on the tagged information, classifying the electrocardiogram data as a first group having a high relative value; and, when the hospital size does not meet the preset reference size or the patient's property does not match the preset reference property, classifying the electrocardiogram data as a second group having a low relative value.

Alternatively, the electrocardiogram data classified as the first group may be divided into training data and verification data at a preset ratio according to the hospital type of the tagged information.

According to another embodiment of the present disclosure for achieving the above-described object, there is disclosed a computer program stored in a computer-readable storage medium, the computer program performing operations for updating an artificial intelligence model for electrocardiogram reading when executed on one or more processors, wherein the operations include operations of: obtaining electrocardiogram data; classifying the relative value of the electrocardiogram data using at least one of a first value determination, a second value determination, and a third value determination based on preset reference value ranges by analyzing the electrocardiogram data; and combining pieces of electrocardiogram data whose relative values have been classified to re-train a previously constructed first machine learning model and to train one or more new second machine learning models.

According to still another embodiment of the present disclosure for achieving the above-described object, there is disclosed a computing device for updating an artificial intelligence model for electrocardiogram reading, the computing device including: a processor including at least one core; and memory including program codes executable on the processor; wherein the processor, as the program codes are executed: obtains electrocardiogram data; classifies the relative value of the electrocardiogram data using at least one of a first value determination, a second value determination, and a third value determination based on preset reference value ranges by analyzing the electrocardiogram data; and combines pieces of electrocardiogram data whose relative values have been classified to re-train a previously constructed first machine learning model and to train one or more new second machine learning models.

### Advantageous Effects

The method of updating an artificial intelligence model for electrocardiogram reading according to one embodiment of the present disclosure may re-train a previously constructed machine learning model or train a new machine learning model using training data based on electrocardiogram data having high relative value, so that the output accuracy of the artificial intelligence model can be further increased, thereby providing a more accurate electrocardiogram reading service.

In addition, the present disclosure may automatically classify newly added electrocardiogram data according to relative value, may generate training data using data whose relative values have been classified to re-train or train artificial intelligence models, and may select a model having the highest output accuracy from among the re-trained or trained artificial intelligence models, thereby providing the effect of reducing the effort and time required for updating, operating, and managing an artificial intelligence model and the effect of efficiently developing and distributing an artificial intelligence model.

### Description of Drawings

FIG. 1 is a block diagram of a computing device according to one embodiment of the present disclosure;
FIG. 2 is a block diagram of a system according to one embodiment of the present disclosure;
FIG. 3 is a flowchart showing a method of updating an artificial intelligence model for electrocardiogram reading according to one embodiment of the present disclosure;
FIG. 4 is a diagram illustrating a process of selecting a model for an electrocardiogram reading service according to one embodiment of the present disclosure;
FIG. 5 is a flowchart illustrating a process of performing a first value analysis process according to one embodiment of the present disclosure;
FIG. 6 is a flowchart illustrating a process of performing a second value analysis process according to one embodiment of the present disclosure; and
FIG. 7 is a flowchart illustrating a process of performing a third value analysis process according to one embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "x uses a or b" should be understood to mean one of the natural inclusive substitutions. For example, unless otherwise specified herein or the meaning is not clear from the context, the clause "x uses a or b" may be interpreted as any one of a case where x uses a, a case where x uses b, and a case where x uses both a and b.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "n-th (n is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computerrelated entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The "data" used herein may include an "image." The term "image" used herein may refer to multidimensional data composed of discrete image elements. In other words, the "image" may be understood as a term referring to a digital representation of an object that can be seen by the human eye. For example, the term "image" may refer to multidimensional data composed of elements corresponding to pixels in a twodimensional image. The term "image" may refer to multidimensional data composed of elements corresponding to voxels in a three-dimensional image.

The term "block" used herein may be understood as a set of components classified based on various criteria such as type, function, etc. Accordingly, the components classified as each "block" may be changed in various manners depending on the criteria. For example, a neural network "block" may be understood as a set of neural networks including one or more neural networks. In this case, it can be assumed that the neural networks included in the neural network "block" perform the same specific operations. The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

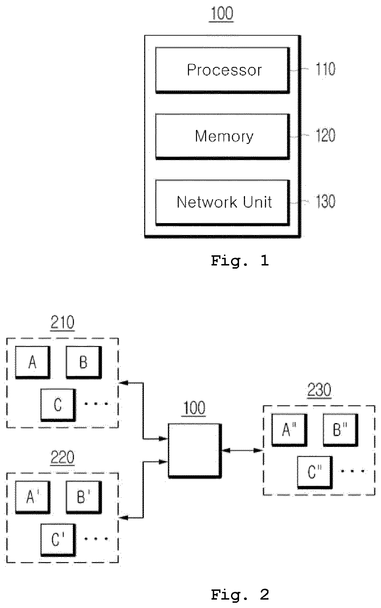

FIG. 1 is a block diagram of a computing device according to one embodiment of the present disclosure.

The computing device 100 according to the one embodiment of the present disclosure may be a hardware device or a part of a hardware device that performs the comprehensive processing and operation of data, or may be a software-based computing environment that is connected to a communication network. For example, the computing device 100 may be a server that is a principal agent performing an intensive data processing function and sharing resources, or may be a client that shares resources through interaction with a server or a specific terminal. Furthermore, the computing device 100 may be a cloud system in which pluralities of servers and clients comprehensively process data while interacting with each other. Furthermore, the computing device 100 may be a component of a medical robot. Since the above-described description is only one example related to the type of the computing device 100, the type of the computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to one embodiment of the present disclosure may be understood as a component unit including hardware and/or software for performing computing operation. For example, the processor 110 may read a computer program and perform data processing for machine learning. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may train or re-train an artificial intelligence model for electrocardiogram reading based on electrocardiogram data. For example, the processor 110 may train or re-train an artificial intelligence model to estimate electrocardiogram reading results such as diagnosis identification and patient classification based on biological data, including information such as gender, age, weight, height, and/or the like, together with electrocardiogram data. The processor 110 may perform the operation of representing at least one neural network block included in a machine learning model during a process of training the artificial intelligence model.

The processor 110 may re-train a previously constructed first machine learning model by using only the highly reliable electrocardiogram data of obtained electrocardiogram data as training data, and may train one or more new second machine learning models. Furthermore, the output accuracy of the artificial intelligence model may be increased by updating a usage model by comparing the re-trained first machine learning model and the trained new second machine learning models. That is, the processor 110 may periodically update and optimize a model to be used for electrocardiogram reading by re-training the existing model via data having a high training value or by training the new models and comparing them with the existing model.

In addition to the examples described above, the types of medical data including electrocardiogram data and the output of the neural network model may be configured in various manners within a range understandable to those skilled in the art based on the content of the present disclosure.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data required for the processor 110 to perform operations, the combination of data, and program codes executable on the processor 110. For example, the memory 120 may store medical data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, and/or the like

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data required for the processor 110 to perform operations through wired/wireless communication with any system or client or the like. Furthermore, the network unit 130 may transmit data generated through the operation of the processor 110 through wired/wireless communication with any system or client or the like. For example, the network unit 130 may receive medical data through communication with a database within a hospital environment, a cloud server configured to perform tasks such as the standardization of medical data, a computing device, or the like. The network unit 130 may transmit the output data of the neural network model, intermediate data and processed data obtained from the operation process of the processor 110, and/or the like through communication with the above-described database, server, or computing device.

FIG. 2 is a block diagram of a system according to one embodiment of the present disclosure.

Referring to FIG. 2, a system according to one embodiment of the present disclosure may include a computing device 100 configured to process operation processes for updating an artificial intelligence model, and a plurality of clients 210, 220, and 230 configured to assist the operation processes processed by the computing device 100 through communication with the computing device 100. In FIG. 2, the clients 210, 220, and 230 are illustrated as entities independent of the computing device 100, but the clients 210, 220, and 230 may be components included in the computing device 100.

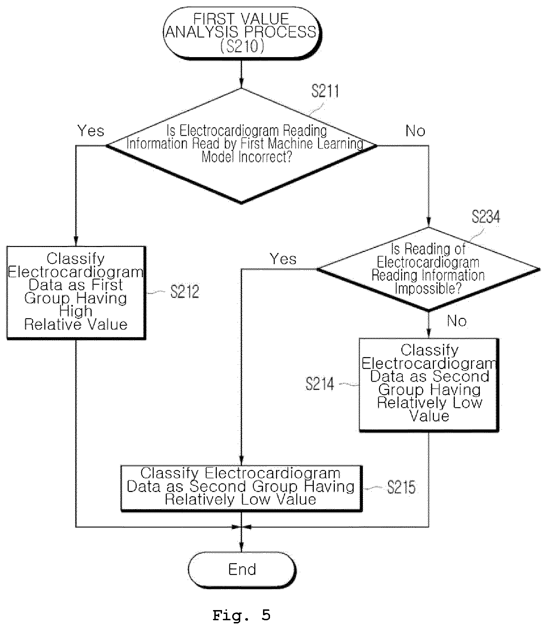

The computing device 100 classifies the relative value of electrocardiogram data using at least one of a first value determination, a second value determination, and a third value determination based on preset reference value ranges by analyzing the electrocardiogram data. The computing device 100 may include an incorrect answer extraction module configured to perform a first value analysis process for the first value determination, a patient extraction module configured to perform a second value analysis process for the second value determination, and a property tagging module configured to perform a third value analysis process for the third value determination. The first value analysis process, the second value analysis process, and the third value analysis process will be described in conjunction with FIGS. 5 to 7 below.

The above-described modules are only examples intended to describe the present invention, and are not limited thereto and may be implemented in various modifications. Furthermore, the above-described modules are stored in the memory 120 as computer-readable storage media that can be controlled by the processor 110. Furthermore, at least some of the above-described modules may each be implemented as software, firmware, hardware, or a combination of at least two or more thereof, and may each include a module, a program, a routine, an instruction set, or a process for performing at least one function.

The first client 210 may be understood as a client including experts (e.g., one or more data labelers, and one or more readers) who review and label the electrocardiogram data obtained for an electrocardiogram reading service.

The second client 220 may be understood as a client including a group of developers (artificial intelligence experts, etc.) who actually conduct research and design for a fine-tuning method for a previously constructed artificial intelligence model through the development of new artificial intelligence technology and data analysis.

The third client 230 may be understood as a client including the operators of the electrocardiogram reading service who distribute models exhibiting the best performance among the artificial intelligence models satisfying the criteria set according to the type of service and are in charge of tasks of updating the artificial intelligence models.

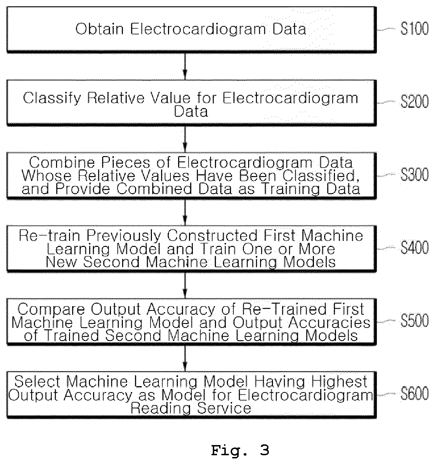

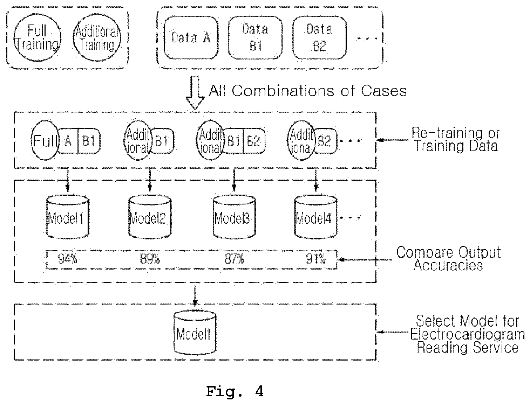

FIG. 3 is a flowchart showing a method of updating an artificial intelligence model for electrocardiogram reading according to one embodiment of the present disclosure, and FIG. 4 is a diagram illustrating a process of selecting a model for an electrocardiogram reading service according to one embodiment of the present disclosure.

Referring to FIG. 3, in the method of updating an artificial intelligence model for electrocardiogram reading that is performed by the computing device 100 including at least one processor, step S100 of obtaining raw electrocardiogram data may be performed first.

The electrocardiogram data may be obtained directly through measurement via an electrocardiogram measurement device, or may be obtained through network communication from an electrocardiogram measurement device. More specifically, the electrocardiogram data is obtained via a plurality of electrodes placed on a specific surface of the body, and a heart condition is evaluated by measuring the voltages between the electrodes.

The electrocardiogram measurement device may employ various devices capable of measuring electrocardiograms, such as wearable devices capable of being worn on a user's body and measuring and collecting various health indicators such as heart rate, body fat percentage, blood pressure, and/or the like, and electrocardiogram kiosks. In this case, the electrocardiogram measurement device may measure electrocardiograms by using various electrode combinations such as not only a single-lead method using a wearable device such as a wristwatch or patch, but also a 12-lead method, a 6-lead method, and the like. It is also desirable that the electrocardiogram measurement time is increased or decreased and then set according to the signal to be obtained.

In general, a standard electrocardiogram measurement method includes 12 leads (channels), each of which measures a specific potential difference. However, a patient needs to have at least 10 electrodes attached to their skin at a medical facility, and thus, it is practically difficult to maintain all 12 leads, so that many medical professionals and cardiologists are actually utilizing a reduced channel system.

The computing device 100 analyzes the electrocardiogram data and classifies the relative value of the electrocardiogram data by using at least one of the first value determination, the second value determination, and the third value determination based on the preset reference value ranges in step S200.

The computing device 100 may generate training data by combining pieces of electrocardiogram data whose relative values are classified in step S300, and may re-train a previously constructed first machine learning model using the training data generated in this manner and train one or more new second machine learning models in step S400. The training data may be data processed as a pair of input data and label data, with electrocardiogram data used as the input data and electrocardiogram reading result data for the corresponding electrocardiogram data used as the label data. In this case, the electrocardiogram reading result data may include expert reading information obtained by an expert terminal and electrocardiogram reading information obtained via an artificial intelligence model.

Furthermore, the above-described re-training or training step S400 may further include the step of inputting biological data, including at least one of age, gender, weight, height, and/or the like, together with electrocardiogram data, as variables affecting the characteristics of heart disease to the first machine learning model and the second machine learning models, so that the first machine learning model and the second machine learning models can be trained based on the correlations between the biological data and the heart disease.

The computing device 100 may compare the output accuracy of the re-trained first machine learning model and the trained second machine learning models in step S500, and may select a machine learning model having the highest output accuracy as a model for an electrocardiogram reading service in step S600.

As shown in FIG. 4, the computing device 100 may set existing cumulative electrocardiogram data A and electrocardiogram data, classified as having a high relative value, as a first group B1, and may also set electrocardiogram data, classified as having a low relative value, as a second group B2. The computing device 100 may also subdivide each group or add a new group according to the reference value ranges of relative values.

In general, the sizes of hospitals are diverse, such as primary hospitals (neighborhood clinics), secondary hospitals (semi-general hospitals), and tertiary hospitals (university hospitals), and the types of patients (mothers, children, adults, heart patients, preoperative patients, and plastic surgery patients) are also diverse. Accordingly, the computing device 100 may provide an artificial intelligence model with additional data, such as B1-1 (for clinics), B1-2 (for general hospitals), or B1-3 (for plastic surgery), set according to the situation of the hospital that has requested an artificial intelligence model service, thereby providing the development of a customized artificial intelligence model and a customized electrocardiogram reading service for each hospital.

In this manner, the second client 220 may develop and provide at least one artificial intelligence model using various learning methods and various setting values. In this case, the second client 220 may be an entity independent of the computing device 100, or may be one component included in the computing device 100.

As an example, the second client 220 may set an artificial intelligence, having the same structure as the first machine learning model, as a basic model, and may generate new second machine learning models, such as model 1, model 2, and model 3, trained with different types of training data using the basic model. In this case, the training data may be a dataset generated using all possible cases that can be obtained by combining a plurality of learning methods, the first group, and the training data of the group.

In this case, the learning method may include a full learning method and an additional learning method, and the training data may include data A, data B1, and data B2. Accordingly, 14 datasets may be generated by using the number of combinations of learning methods and the number of combinations of training data.

The full learning method is to input existing data A and additionally generated electrocardiogram data B1 + B2 to an untrained artificial intelligence model at once and learn them, and the additional learning method may be to add additionally generated electrocardiogram data B1 or B2 to an artificial intelligence model trained with existing data A and learn it partially.

Accordingly, the second client 220 may generate 14 machine learning models using 14 datasets, and may train each of 14 machine learning models using the training dataset.

When the training of the 14 machine learning models is completed, the computing device 100) extracts the output accuracy for each of the machine learning models using verification data. For example, assuming that the output accuracy of model 1 is 94%, the output accuracy of model 2 is 89%, the output accuracy of model 3 is 87%, and the output accuracy of model 4 is 91%, the computing device 100 may select model 1 as the model for the electrocardiogram reading service.

In this case, the computing device 100 may provide an alarm function including information about a machine learning model having the highest output accuracy so that the third client 230, i.e., the operator of the electrocardiogram reading service, may update the model for the electrocardiogram reading service via the alarm function.

The selection of the model for the electrocardiogram reading service is performed when a preset selection trigger condition is satisfied. The selection trigger condition may be either a case where the number of machine learning models whose output accuracy exceeds a first criterion exceeds a second criterion or a case where the number of pieces of electrocardiogram data determined to have a disease exceeds a third criterion.

When the third client 230 selects a new second machine learning model that will replace a previously constructed first machine learning model, it may select the new second machine learning model when the selection trigger condition is satisfied, rather than making determination based on a preset time.

To this end, the computing device 100 may provide alarm information when the number of machine learning models whose output accuracy exceeds the first criterion out of the models that have been continuously trained with newly generated training data exceeds the second criterion, and may provide alarm information when the number of pieces of electrocardiogram data determined to have a disease exceeds the third criterion. The computing device 100 may include information about the machine learning model having the highest output accuracy in the alarm information so that one of the previously constructed machine learning models and the new machine learning models can be selected.

FIG. 5 is a flowchart illustrating a process of performing a first value analysis process according to one embodiment of the present disclosure.

More specifically, FIG. 5 shows one embodiment of step S200 of FIG. 3 in more detail. Step S200 of classifying the relative value of electrocardiogram data may use at least one of the first value determination, the second value determination, and the third value determination based the preset reference value ranges. FIG. 5 illustrates a first value analysis process S210 of classifying the relative value of electrocardiogram data by using the first value determination that determines the value of the electrocardiogram data by comparing the expert reading information read by the expert terminal for the expert's indepth reading of electrocardiogram data with the electrocardiogram reading information read via the first machine learning model.

In the first value analysis process S210, the computing device 100 determines whether the electrocardiogram reading information read via the first machine learning model is incorrect in step S211. When the electrocardiogram reading information is incorrect, the computing device 100 classifies the electrocardiogram data as a first group having a high relative value in step S212.

When the electrocardiogram reading information is not incorrect and electrocardiogram reading is not in an impossible state attributable to an error signal caused by a noise signal or missing value included in the electrocardiogram data in step S213, the computing device 100 classifies electrocardiogram data as a second group having a relatively low value in step S214.

That is, data for which electrocardiogram reading information read via the first machine learning model is incorrect may be understood as data having a high value that can increase the accuracy of the model and improve the model itself compared to data for which electrocardiogram reading information read via the first machine learning model is not incorrect.

In this case, when the electrocardiogram reading information is not an incorrect answer and the electrocardiogram data cannot be read in step S213, the computing device 100 discards the corresponding electrocardiogram data not to use it as data for re-training or training in step S215.

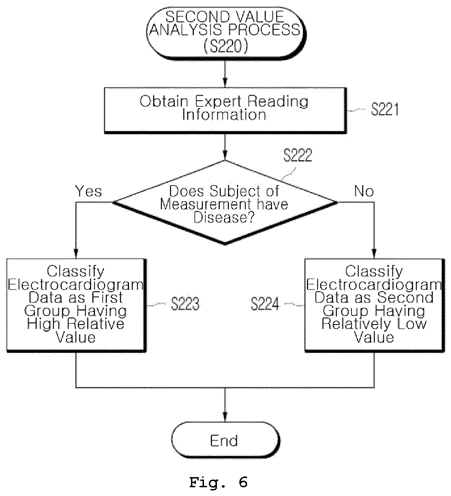

FIG. 6 is a flowchart illustrating a process of performing a second value analysis process according to one embodiment of the present disclosure.

More specifically, FIG. 6 illustrates one embodiment of step S200 of FIG. 3 in more detail. FIG. 6 illustrates a second value analysis process S220 that analyzes whether a subject from whom the electrocardiogram data was measured has a disease and classifies the value of the electrocardiogram data by using the second value determination for determining the value of electrocardiogram data.

In the second value analysis process S220, the computing device 100 obtains expert reading information for electrocardiogram data from the expert terminal in step S221, and determines whether a subject from whom the electrocardiogram data was measured has a disease based on the obtained expert reading information in step S222. When the subject from whom the electrocardiogram data was measured has a disease, the computing device 100 classifies the electrocardiogram data as a first group having a high relative value in step S223. When the subject from whom the electrocardiogram data was measured is determined not to have a disease, the computing device 100 classifies the electrocardiogram data as a second group having a low relative value in step S224.

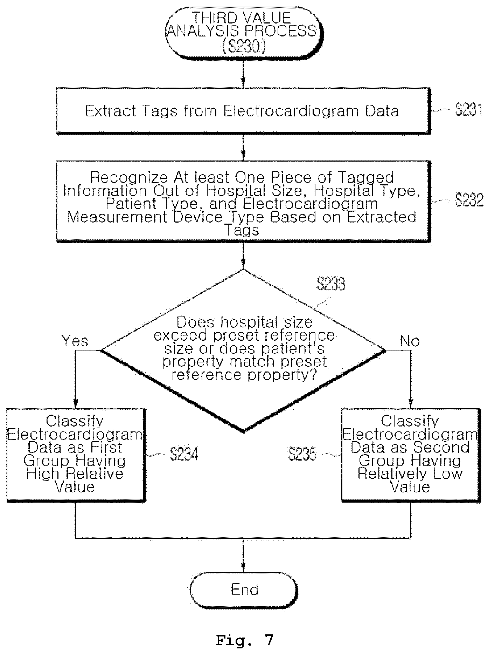

FIG. 7 is a flowchart illustrating a process of performing a third value analysis process according to one embodiment of the present disclosure.

More specifically, FIG. 7 shows one embodiment of step S200 of FIG. 3 in more detail. FIG. 7 illustrates a third value analysis process S230 that classifies the value of electrocardiogram using the third value determination for determining the value of electrocardiogram data based on tags included in the electrocardiogram data.

In the third value analysis process S230, the computing device 100 extracts tags from the electrocardiogram data via a tag interface for analyzing tags included in the electrocardiogram data in step S231.

The computing device 100 recognizes at least one piece of tagged information out of the hospital size, the hospital type, the patient type, and the electrocardiogram measurement device type based on the extracted tags in step S232.

When the hospital size meets a preset reference size or the patient's property match preset reference property based on the tagged information in step S233, the computing device 100 may classify the electrocardiogram data as a first group having a high relative value in step S234. In this case, the fact that the hospital size meets a reference size may be understood as the size of the hospital in which the machine learning model will be used corresponding to the size of the hospital in which the training data was measured. Furthermore, the patient's property may be understood as corresponding to the type of patient whose diagnosis or treatment is medically distinguished, such as a pregnant woman, a critically ill patient, a child, or an adult. In other words, the fact that the patient's property matches a preset reference property may be understood as checking whether a tag such as a pregnant woman, a critically ill patient, a child, or an adult is accurately attached. When the hospital size does not meet the reference size or the patient's property does not match the preset reference feature, the computing device 100 may classify the electrocardiogram data as a second group having a low relative value in step S235. In this case, the reference size of the hospital size may correspond to a primary hospital, but may be set in various manners depending on the purpose of use.

Meanwhile, the computing device 100 may divide the electrocardiogram data, classified as the first group, into training data and verification data at a preset ratio according to the hospital type of the tagged information. For example, when the hospital type in the tagged information includes a tag for a specialized hospital related to the heart, including a heart disease center or a cancer center, the proportion of verification data for the corresponding electrocardiogram data may be set to be higher than that of training data. Through this division, the problem of data bias in which the data of a specific hospital is concentrated on training or verification may be prevented.

In this manner, in the present disclosure, training data may be generated using electrocardiogram data whose relative values have been classified and an artificial intelligence model updated using such training data is used, so that the output accuracy of the artificial intelligence model can be increased, and thus, the time for medical staff to read the electrocardiogram can be saved. Furthermore, only highly reliable electrocardiogram data is obtained through classification and used as training data instead of using all pieces of electrocardiogram data as training data, so that the overload of the artificial intelligence can be prevented.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of updating an artificial intelligence model for electrocardiogram reading, the method being performed by a computing device including at least one processor, the method comprising:
obtaining electrocardiogram data;
classifying a relative value of the electrocardiogram data using at least one of a first value determination, a second value determination, and a third value determination based on preset reference value ranges by analyzing the electrocardiogram data; and
combining pieces of electrocardiogram data whose relative values have been classified to re-train a previously constructed first machine learning model and to train one or more new second machine learning models.

2. The method of claim 1, further comprising selecting a machine learning model having a highest output accuracy as a model for an electrocardiogram reading service by comparing an output accuracy of the re-trained first machine learning model and output accuracies of the trained second machine learning models.

3. The method of claim 2, further comprising providing an alarm function including information about the machine learning model having the highest output accuracy so that an operator of the electrocardiogram reading service can update the model for the electrocardiogram reading service.

4. The method of claim 2, wherein the selection of the model for the electrocardiogram reading service is performed when a preset selection trigger condition is satisfied.

5. The method of claim 4, wherein the selection triggering condition is any one of a case where a number of machine learning models whose output accuracy exceeds a first criterion exceeds a second criterion and a case where a number of pieces of electrocardiogram data determined to have a disease exceeds a third criterion.

6. The method of claim 1, wherein the first value determination includes a first value analysis process of determining the value of the electrocardiogram data by comparing expert reading information read by an expert terminal with electrocardiogram reading information read via the first machine learning model.

7. The method of claim 6, wherein the first value analysis process includes:
a process of determining whether the electrocardiogram reading information read via the first machine learning model is incorrect; and
a process of classifying the relative value of the electrocardiogram data depending on whether the electrocardiogram reading information is incorrect.

8. The method of claim 7, wherein the process of classifying the relative value of the electrocardiogram data depending on whether the electrocardiogram reading information is incorrect includes:
when the electrocardiogram reading information is incorrect, classifying the electrocardiogram data as a first group having a high relative value; and
when the electrocardiogram reading information is not incorrect, classifying the electrocardiogram data as a second group having a low relative value.

9. The method of claim 8, wherein the process of classifying the relative value of the electrocardiogram data depending on whether the electrocardiogram reading information is incorrect includes, when the electrocardiogram reading information is not incorrect and electrocardiogram reading is impossible due to an error signal caused by a noise signal or missing value included in the electrocardiogram data, discarding the electrocardiogram data not to be used as data for re-training or training.

10. The method of claim 1, wherein the second value determination includes a second value analysis process that determines the value of the electrocardiogram data by analyzing whether a subject from whom the electrocardiogram data was measured has a disease.

11. The method of claim 10, wherein the second value analysis process includes, based on expert reading information read by an expert terminal:
when it is determined that the subject from whom the electrocardiogram data was measured has a disease, classifying the electrocardiogram data as a first group having a high relative value; and
when it is determined that the subject from whom the electrocardiogram data was measured does not have a disease, classifying the electrocardiogram data as a second group having a low relative value.

12. The method of claim 1, wherein the third value determination includes a third value analysis process that determines the value of the electrocardiogram data based on a tag included in the electrocardiogram data.

13. The method of claim 12, wherein the third value analysis process includes:
extracting one or more tags from the electrocardiogram data through a tag interface for analyzing tags included in the electrocardiogram data;
recognizing at least one tagged information out of a hospital size, a hospital type, a patient type, and an electrocardiogram measurement device type based on the extracted tags; and
classifying the relative value of the electrocardiogram data based on the recognized information.

14. The method of claim 13, wherein classifying the relative value of the electrocardiogram data based on the recognized information includes:
when the hospital size meets a preset reference size or the patient's property matches a preset reference property based on the tagged information, classifying the electrocardiogram data as a first group having a high relative value; and
when the hospital size does not meet the preset reference size or the patient's property does not match the preset reference property, classifying the electrocardiogram data as a second group having a low relative value.

15. The method of claim 14, wherein the electrocardiogram data classified as the first group is divided into training data and verification data at a preset ratio according to the hospital type of the tagged information.

16. A computer program stored in a computer-readable storage medium, the computer program performing operations for updating an artificial intelligence model for electrocardiogram reading when executed on one or more processors, wherein the operations comprise operations of:
obtaining electrocardiogram data;
classifying a relative value of the electrocardiogram data using at least one of a first value determination, a second value determination, and a third value determination based on preset reference value ranges by analyzing the electrocardiogram data; and
combining pieces of electrocardiogram data whose relative values have been classified to re-train a previously constructed first machine learning model and to train one or more new second machine learning models.

17. A computing device for updating an artificial intelligence model for electrocardiogram reading, the computing device comprising:
a processor including at least one core; and
memory including program codes executable on the processor;
wherein the processor, as the program codes are executed:
obtains electrocardiogram data;
classifies a relative value of the electrocardiogram data using at least one of a first value determination, a second value determination, and a third value determination based on preset reference value ranges by analyzing the electrocardiogram data; and
combines pieces of electrocardiogram data whose relative values have been classified to re-train a previously constructed first machine learning model and to train one or more new second machine learning models.
